# EUROPEAN PATENT APPLICATION

(11) **EP 4 633 196 A1**
(43) Date of publication of application: **15.10.2025**
(21) Application number: 25151465.9
(22) Date of filing: 13.01.2025
(51) Int. Cl.: H04R 3/12, H04S 5/00

(54) **SOUND ADJUSTMENT DEVICE**

(30) Priority: 12.04.2024 TW 113113663
(71) Applicant: Cotron Corporation, Taipei City 111 (TW)
(72) Inventor: YANG, Bill, 111 Taipei City (TW)
(74) Representative: Becker, Eberhard

(57) **Abstract**

A sound adjustment device (100) including a background sound source (110), an external sound source receiver (120), a frequency adjuster (130), a mixer (140), and an output port (150). A background audio signal (B10) provided by the background sound source (110) is a mono audio signal. The external sound source receiver (120) is used to receive an external audio signal (E10). The frequency adjuster (130) is used to receive and adjust the background audio signal (B10) into a left ear background audio signal (B12) and a right ear background audio signal (B14). A first frequency of the left ear background audio signal (B12) and a second frequency of the right ear background audio signal (B14) have a frequency difference. The mixer (140) is used to mix the external audio signal (E10) and the left ear background audio signal (B12) into a left ear audio signal (L10), and to mix the external audio signal (E10) and the right ear background audio signal (B14) into a right ear audio signal (R10).

## Description

### BACKGROUND

### Technical Field

The disclosure relates to an adjustment device, and in particular to a sound adjustment device.

### Description of Related Art

Today, with the rapid advancement of technology and the booming of audio-visual content, people use headphones more often to listen to music or enjoy audio-visual content without disturbing other people nearby. However, how to improve the experience of using headphones has been a topic that headphone manufacturers constantly work on.

### SUMMARY

The disclosure provides a sound adjustment device, through which a user perceives binaural beats.

A sound adjustment device of the disclosure includes a background sound source, an external sound source receiver, a frequency adjuster, a mixer, and an output port. The background sound source is used to provide a background audio signal. The background audio signal is a mono audio signal. The external sound source receiver is used to receive an external audio signal. The frequency adjuster is used to receive the background audio signal and adjust the background audio signal into a left ear background audio signal and a right ear background audio signal. A first frequency of the left ear background audio signal and a second frequency of the right ear background audio signal have a frequency difference. The mixer is used to mix the external audio signal and the left ear background audio signal into a left ear audio signal. The mixer is also used to mix the external audio signal and the right ear background audio signal into a right ear audio signal. The output port is used to output the left ear audio signal to a left ear speaker, and to output the right ear audio signal to a right ear speaker.

In an embodiment of the sound adjustment device, when the mixer performs mixing, the mixer further adjusts a volume of the left ear background audio signal of the left ear audio signal, and adjusts a volume of the right ear background audio signal of the right ear audio signal.

In an embodiment of the sound adjustment device, the external sound source receiver is a wireless receiver.

In an embodiment of the sound adjustment device, the external audio signal is a stereo audio signal including a left ear external audio signal and a right ear external audio signal. The mixer is used to mix the left ear external audio signal and the left ear background audio signal into the left ear audio signal. The mixer is further used to mix the right ear external audio signal and the right ear background audio signal into the right ear audio signal.

In an embodiment of the sound adjustment device, the background sound source generates the background audio signal using an algorithm.

In an embodiment of the sound adjustment device, the background audio signal is a rain sound audio signal.

In an embodiment of the sound adjustment device, the background audio signal is pre-recorded and stored in the background sound source.

Based on the above, in the sound adjustment device of the disclosure, sound waves received in both ears have a frequency difference, thereby providing the user with corresponding effects in addition to hearing the sound waves.

### BRIEF DESCRIPTION OF THE DRAWINGS

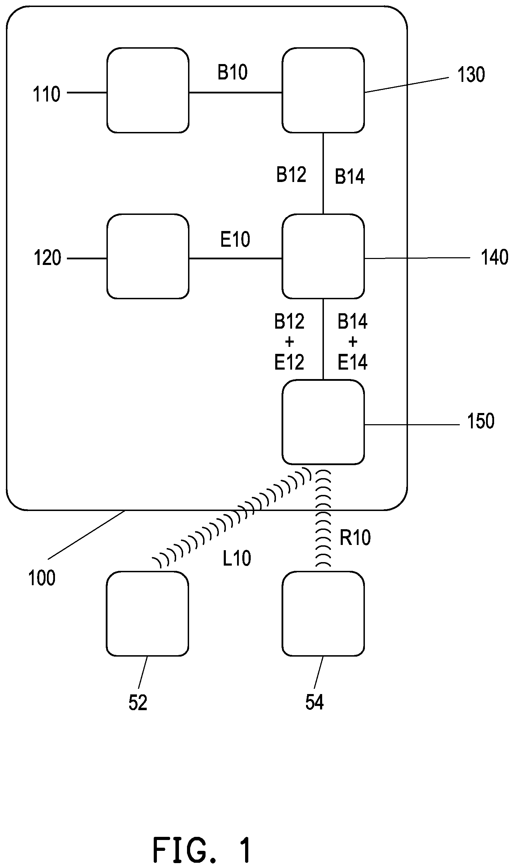

FIG. 1 is a schematic circuit block diagram of a sound adjustment device in an embodiment of the disclosure.

### DESCRIPTION OF THE EMBODIMENTS

FIG. 1 is a schematic circuit block diagram of a sound adjustment device in an embodiment of the disclosure. Referring to FIG. 1, a sound adjustment device 100 in this embodiment includes a background sound source 110, an external sound source receiver 120, a frequency adjuster 130, a mixer 140, and an output port 150. The background sound source 110 is used to provide a background audio signal B10. The background audio signal B10 is a mono audio signal. The frequency adjuster 130 is used to receive the background audio signal B10 and adjust the background audio signal B10 into a left ear background audio signal B12 and a right ear background audio signal B14. A first frequency of the left ear background audio signal B12 and a second frequency of the right ear background audio signal B14 have a frequency difference.

Since the background audio signal B10 is a mono audio signal, a part of the signal to be output to a left ear speaker 52 is the same as a part of the signal to be output to a right ear speaker 54. Even if the frequency adjuster 130 adjusts a frequency of the background audio signal B10 and divides the background audio signal B10 into the left ear background audio signal B12 and the right ear background audio signal B14, the sound that the user heard is not significantly affected. Moreover, the background audio signal B10 is not the main focus of the user in the first place, which reduces the impact on the listening experience.

The external sound source receiver 120 is used to receive an external audio signal E10. The mixer 140 is used to mix the external audio signal E10 and the left ear background audio signal B12 into a left ear audio signal L10. The mixer 140 is also used to mix the external audio signal E10 and the right ear background audio signal B14 into a right ear audio signal R10. That is, a frequency of the external audio signal E10 is not adjusted by the frequency adjuster 130. Only the frequency of the background audio signal B10 is adjusted by the frequency adjuster 130. Thus, the external audio signal E10 can be faithfully generated from the left ear speaker 52 and the right ear speaker 54 and provided to the user without modulation.

After mixing by the mixer 140, the output port 150 outputs the left ear audio signal L10 to the left ear speaker 52 and outputs the right ear audio signal R10 to the right ear speaker 54.

Brainwaves refer to the electrical oscillations generated through nerve cell activity in the human brain, and may also be described as the rhythm of brain cell activity. The human brain produces brainwaves at all times. Classified by frequency, brainwaves at least include beta waves, alpha waves, theta waves, delta waves, and gamma waves. Generally, when a person is in the third stage of non-rapid eye movement sleep, the brainwaves are usually delta waves (a frequency of which falls between 0.5Hz and 4Hz). When a person is in a state of deep sleep, dreaming, deep meditation, subjective intensity, and the like, the brainwaves are usually theta waves (a frequency of which falls between 4Hz and 8Hz). When a person is in a state of drowsiness before sleep, vague consciousness, sudden insight, focused awareness but with a relaxed body and mind, and the like, the brainwaves are usually alpha waves. When a person is in a state of relaxation but focused, processing previously received information, and the like, the brainwaves are usually beta waves. However, research also shows that when the human brain is induced to generate different brainwaves, the state of the person may in turn be affected. For example, when the brain is induced to produce alpha waves, the person may also be brought into a state of sudden insight, focused awareness but with a relaxed body and mind, and the like.

By adjusting the frequency of the background audio signal B10 through the frequency adjuster 130, the frequency difference between the first frequency of the left ear background audio signal B12 and the second frequency of the right ear background audio signal B14 is set to induce the brain of the user to generate beta waves, alpha waves, theta waves, delta waves, gamma waves, or other brainwaves so as to lead the user into the aforementioned corresponding states, thereby achieving effects such as enhanced focus, relaxation for sleep, stress relief, getting into meditation, or the like.

In this embodiment, the frequency difference automatically and circularly changes over time. That is, the frequency difference is not fixed and unchangeable. Moreover, no human intervention is required as the frequency difference automatically changes according to a preset means. In addition, the changing means of the frequency difference is pre-set and cannot be adjusted or selected by the user. Alternatively, there may be multiple changing means for the user to select. In addition, before the changing means of the frequency difference is determined, the physiological state of the user is not detected, which means the changing means of the frequency difference does not vary according to the physiological state of the user.

For example, the frequency difference may automatically and circularly change between 8Hz and 12Hz stepwise, with each change in the frequency difference being 0.1Hz. A same frequency difference is maintained for 10 seconds after each change. The automatic, stepwise, and circular change of the frequency difference between 8Hz and 12Hz refers to, for example, a gradual change from 8Hz to 12Hz and then back from 12Hz to 8Hz, or, for example, a repeated gradual change from 8Hz to 12Hz, or, for example, a repeated gradual change from 12Hz to 8Hz.

In this embodiment, the frequency difference may automatically and circularly change between 0.5Hz and 4Hz to induce delta waves. The frequency difference may automatically and circularly change between 4Hz and 8Hz to induce theta waves. The frequency difference may automatically and circularly change between 8Hz and 12Hz to induce alpha waves. The frequency difference may automatically and circularly change between 12Hz and 20Hz to induce beta waves. The frequency difference may automatically and circularly change between 25Hz and 40Hz to induce gamma waves. In addition, in this embodiment, if limited by an insufficient time period, the frequency difference may simply increase or decrease gradually.

For each user, the brain not only has a better induction effect only on a single frequency difference, but usually has better induction effects on multiple frequency differences. Since the sound adjustment device in this embodiment induces the user with an automatically and circularly changing frequency difference, the sound adjustment device has better induction effects at multiple frequency differences. Although a subject may have a better induction effect with a single input frequency difference, the effect may not last long. Since the sound adjustment device in this embodiment induces the user with an automatically and circularly changing frequency difference, which automatically and circularly changes within a selected range, the sound adjustment device can change to another frequency difference after the user's brain becomes fatigued, thereby generating a better induction effect again. Finally, the sound adjustment device in this embodiment generates a better brainwave induction effect over a longer period.

In this embodiment, when the mixer 140 performs mixing, the mixer 140 further adjusts a volume of the left ear background audio signal B12 of the left ear audio signal L10, and adjusts a volume of the right ear background audio signal B 14 of the right ear audio signal R10. Thus, a brainwave induction effect can be properly generated.

In this embodiment, the external sound source receiver 120 is a wireless receiver that receives the external audio signal E10 wirelessly using Bluetooth, WiFi, FM, or other wireless communication protocols. In other embodiments, the external sound source receiver 120 may also be a wired receiver that receives the external audio signal E10 in a wired manner.

In this embodiment, the external audio signal E10 is a stereo audio signal including a left ear external audio signal E12 and a right ear external audio signal E14. The mixer 140 is used to mix the left ear external audio signal E12 and the left ear background audio signal B12 into the left ear audio signal L10. The mixer 140 is further used to mix the right ear external audio signal E14 and the right ear background audio signal B14 into the right ear audio signal R10. When the external audio signal E10 is a stereo audio signal, since the frequencies of the left ear external audio signal E12 and the right ear external audio signal E14 are not adjusted by the frequency adjuster 130, the external audio signal E10 is faithfully generated from the left ear speaker 52 and the right ear speaker 54 and provided to the user without modulation, providing the user with proper stereo listening quality.

In this embodiment, the background sound source 110 generates the background audio signal B10 using an algorithm. Thus, the background sound source 110 does not require much storage space to store the background audio signal B10. The background audio signal B10 may be a rain sound audio signal or other suitable background audio signals. In this embodiment, the background audio signal B10 may also be pre-recorded and stored in the background sound source 110, providing a more natural listening quality. The left ear speaker 52 and the right ear speaker 54 in this embodiment may be dynamic speakers, balanced armature speakers, bone conduction speakers, or speakers of other forms.

In summary, in the sound adjustment device of the disclosure, the frequency adjuster ensures that the background sound waves received by both ears of the uses have a frequency difference, thereby providing the user with corresponding effects other than the experience of hearing the sound waves. The effects are, for example, enhanced focus, relaxation for sleep, stress relief, getting into meditation, or the like. Moreover, the user can hear the external audio signal with unadjusted frequency, thereby maintaining good listening quality.

## Claims

1. A sound adjustment device (100), comprising:
a background sound source (110), used to provide a background audio signal (B10), wherein the background audio signal (B10) is a mono audio signal;
an external sound source receiver (120), used to receive an external audio signal (E10) from outside;
a frequency adjuster (130), used to receive the background audio signal (B10) and adjust the background audio signal (B10) into a left ear background audio signal (B12) and a right ear background audio signal (B14), wherein a first frequency of the left ear background audio signal (B12) and a second frequency of the right ear background audio signal (B14) have a frequency difference;
a mixer (140), used to mix the external audio signal (E10) and the left ear background audio signal (B12) into a left ear audio signal (L10), and mix the external audio signal (E10) and the right ear background audio signal (B14) into a right ear audio signal (R10); and
an output port (150), used to output the left ear audio signal (L10) to a left ear speaker (52), and output the right ear audio signal (R10) to a right ear speaker (54).

2. The sound adjustment device (100) according to claim 1, wherein when the mixer (140) performs mixing, the mixer (140) further adjusts a volume of the left ear background audio signal (B12) of the left ear audio signal (L10), and adjusts a volume of the right ear background audio signal (B14) of the right ear audio signal (R10).

3. The sound adjustment device (100) according to claim 1, wherein the external sound source receiver (120) is a wireless receiver.

4. The sound adjustment device (100) according to claim 1, wherein the external audio signal (E10) is a stereo audio signal comprising a left ear external audio signal (E12) and a right ear external audio signal (E14), wherein the mixer (140) is used to mix the left ear external audio signal (E12) and the left ear background audio signal (B12) into the left ear audio signal (L10), the mixer (140) further being used to mix the right ear external audio signal (E14) and the right ear background audio signal (B14) into the right ear audio signal (R10).

5. The sound adjustment device (100) according to claim 1, wherein the background sound source (110) generates the background audio signal (B10) using an algorithm.

6. The sound adjustment device (100) according to claim 5, wherein the background audio signal (B10) is a rain sound audio signal.

7. The sound adjustment device (100) according to claim 1, wherein the background audio signal (B10) is pre-recorded and stored in the background sound source (110).
